# EUROPEAN PATENT APPLICATION

(11) **EP 3 939 655 A1**
(43) Date of publication of application: **19.01.2022**
(21) Application number: 20770533.6
(22) Date of filing: 12.03.2020
(51) Int. Cl.: A61P 1/16, A61P 31/20, A61P 35/00, C07H 19/19, A61K 31/7076

(54) **ANTI-HEPATITIS B VIRUS AGENT**

(30) Priority: 14.03.2019 JP 2019046954
(71) Applicant: DAIKIN INDUSTRIES, LTD., Osaka-shi, Osaka 530-8323 (JP); Kagoshima University, Kagoshima-shi, Kagoshima 890-8580 (JP)
(72) Inventor: HAMADA, Tomohito, Osaka-shi, Osaka 530-8323 (JP); IKEUCHI, Hideyuki, Osaka-shi, Osaka 530-8323 (JP); INOO, Kanako, Osaka-shi, Osaka 530-8323 (JP); KISHIKAWA, Yosuke, Osaka-shi, Osaka 530-8323 (JP); IKEDA, Masanori, Kagoshima-shi, Kagoshima 890-8580 (JP); TAKEDA, Midori, Kagoshima-shi, Kagoshima 890-8580 (JP)
(74) Representative: Hoffmann Eitle
(86) International application number: PCT/JP2020/010910
(87) International publication number: WO 2020/184677

(57) **Abstract**

The present disclosure aims to provide an anti-hepatitis B virus agent, and a prophylactic or therapeutic agent for a hepatitis B virus-related disease, each comprising a nucleic acid analogue as an active ingredient.

The above object can be attained by an anti-hepatitis B virus agent, or a prophylactic or therapeutic agent for a hepatitis B virus-related disease, each comprising, as an active ingredient, a compound represented by the following formula (1): 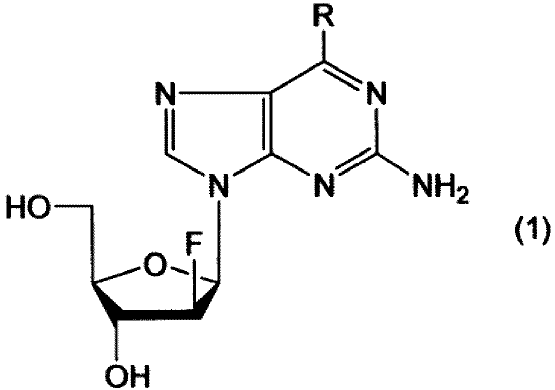 wherein R is a halogen atom, an amino group, a methoxy group, or
a cyano group,
or its prodrug, or a pharmaceutically acceptable salt thereof, or
a solvate thereof.

## Description

### Technical Field

The present disclosure relates to an anti-hepatitis B virus agent, and a prophylactic or therapeutic agent for a hepatitis B virus-related disease, each comprising a nucleic acid analogue as an active ingredient.

### Background Art

As an active ingredient of an anti-hepatoma virus agent and a prophylactic or therapeutic agent for a hepatoma virus-related disease, a nucleic acid analog represented by the following formula: 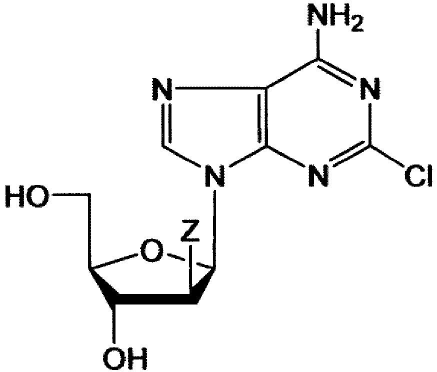
wherein Z is fluorine or hydrogen
is known (Patent Literature 1).

### Citation List

### Patent Literature

PTL 1: WO2017/155082

### Summary of Invention

### Technical Problem

The present disclosure aims to provide an anti-hepatitis B virus agent, and a prophylactic or therapeutic agent for a hepatitis B virus-related disease, each comprising a nucleic acid analogue as an active ingredient.

### Solution to Problem

The present disclosure includes the following embodiments.
Item 1. An anti-hepatitis B virus agent comprising, as an active ingredient, a compound represented by the following formula (1): 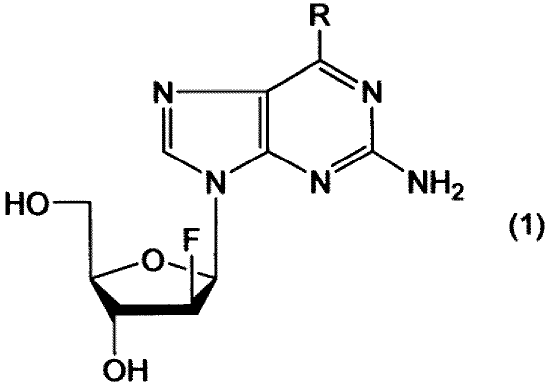
   wherein R is a halogen atom, an amino group, a methoxy group, or a cyano group,
   or its prodrug, or a pharmaceutically acceptable salt thereof, or a solvate thereof.
Item 2. A prophylactic or therapeutic agent for a hepatitis B virus-related disease, comprising, as an active ingredient, a compound represented by the following formula (1): 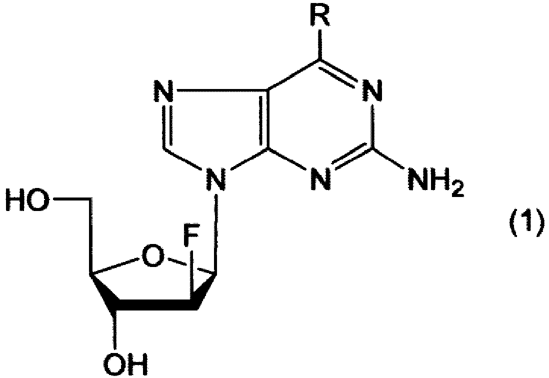
   wherein R is a halogen atom, an amino group, a methoxy group, or a cyano group,
   or its prodrug, or a pharmaceutically acceptable salt thereof, or a solvate thereof.
Item 3. The prophylactic or therapeutic agent according to Item 2, wherein the hepatitis B virus-related disease is one or more diseases selected from the group consisting of hepatitis B, type-B liver cirrhosis, and type-B liver cancer.
Item 4. A compound represented by the following formula (2) or (3) : 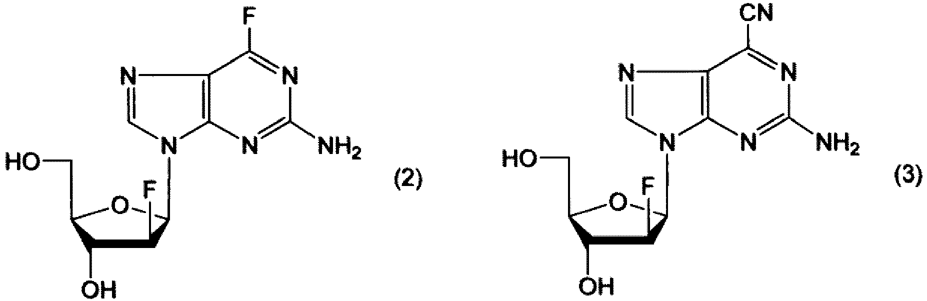 or its prodrug, or a pharmaceutically acceptable salt thereof, or a solvate thereof.
Item 5. A method for producing a compound represented by the following formula (2): 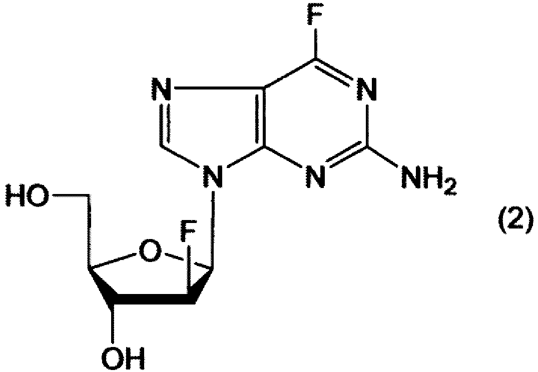 or its prodrug, or a pharmaceutically acceptable salt thereof, or a solvate thereof,
   the method comprising
   step A1 of reacting 2-amino-6-chloropurine with trialkylamine,
   step A2 of reacting the product obtained by the reaction of step A1 with a hydrogen fluoride salt,
   step A of reacting the product obtained by the reaction of step A2 with a compound represented by the following formula (I): 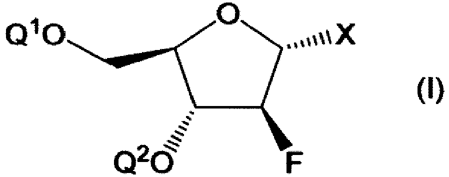
   wherein Q¹ and Q² are the same or different, and each represents a protecting group of a hydroxyl group, and X is bromine or iodine, and
   step B of deprotecting the protecting group of the hydroxyl group of the product obtained by the reaction of step A.
Item 6. A method for producing a compound represented by the following formula (3): 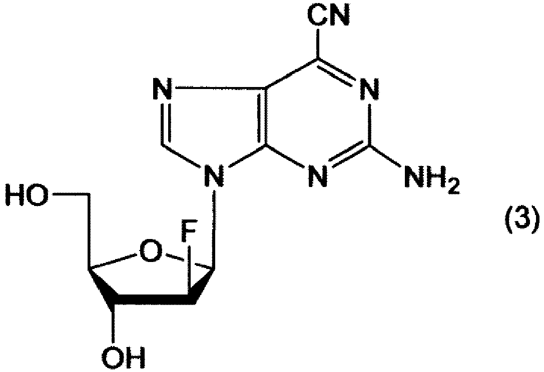 or its prodrug, or a pharmaceutically acceptable salt thereof, or a solvate thereof,
   the method comprising reacting a compound represented by the following formula (4): 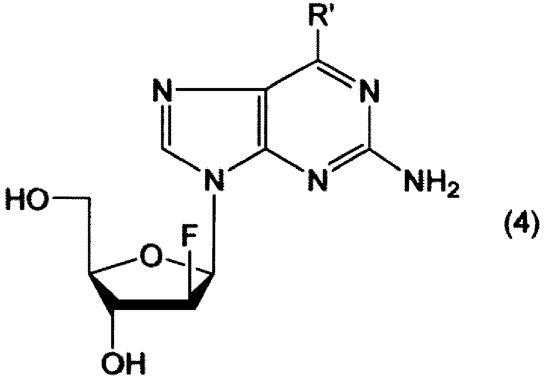
   wherein R' is a halogen atom,
   with a metal cyanide.

The present disclosure further includes the following embodiments.
- The compound represented by formula (1) or its prodrug, or a pharmaceutically acceptable salt thereof, or a solvate thereof, for use as a medicament for preventing or treating a hepatitis B virus-related disease.
- Use of the compound represented by formula (1) or its prodrug thereof, or a pharmaceutically acceptable salt thereof, or a solvate thereof, for the manufacture of a medicament for preventing or treating a hepatitis B virus-related disease.

### Advantageous Effects of Invention

The present disclosure provides an anti-hepatitis B virus agent, and a prophylactic or therapeutic agent for a hepatitis B virus-related disease, each comprising a nucleic acid analogue as an active ingredient.

### Description of Embodiments

The above summary of the present disclosure is not intended to describe each disclosed embodiment or every implementation of the present disclosure.

The description of the present disclosure that follows more specifically provides examples of illustrative embodiments.

In several places throughout the present disclosure, guidance is provided through lists of examples, and these examples can be used in various combinations.

In each instance, the described list serves only as a representative group, and should not be interpreted as an exclusive list.

All of the publications, patents, and patent applications cited herein are incorporated herein by reference in their entirety.

### Terms

Unless otherwise specified, the symbols and abbreviations herein can be understood in the context of the present specification in the meanings commonly used in the art to which the present disclosure belongs.

The terms "contain" and "comprise" as used herein are intended to include "consisting essentially of" and "consisting of."

Unless otherwise specified, the steps, treatments, or operations described herein can be performed at room temperature.

The room temperature referred to herein can mean a temperature in the range of 10 to 40°C.

The notation "Cₙ₋ₘ" (where n and m are each a number) used herein means that the number of carbon atoms is n or more and m or less, as is usually understood by persons skilled in the art.

### Anti-hepatitis B Virus Agent

In the present disclosure, "anti-hepatitis B virus agent" means an agent that delays or inhibits the growth of hepatitis B virus.

Hepatitis B virus may be a strain that is resistant to known anti-hepatitis B virus agents (e.g., Entecavir and Tenofovir).

"Strain that is resistant to known anti-hepatitis B virus agents" means a strain that does not exhibit a growth retardation effect or a growth inhibition effect that normal strains exhibit due to the anti-hepatitis B virus agent, or a strain that exhibits a lower growth retardation effect or lower growth inhibition effect than that of normal strains due to the anti-hepatitis B virus agent.

The anti-hepatitis B virus agent according to one embodiment of the present disclosure is an anti-hepatitis B virus agent comprising, as an active ingredient, a compound represented by the following formula (1): 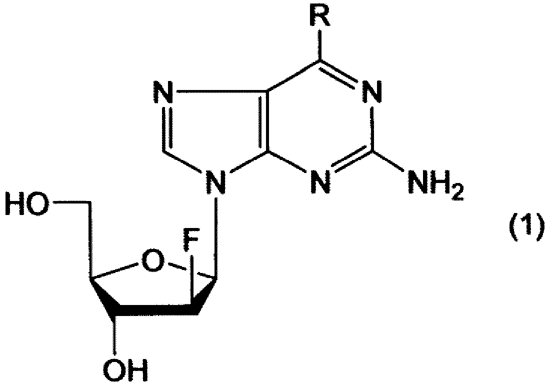
wherein R is a halogen atom, an amino group, a methoxy group, or a cyano group,
or its prodrug, or a pharmaceutically acceptable salt thereof, or a solvate thereof.

### Compound Represented by Formula (1)

In formula (1), examples of the halogen atom represented by R include fluorine, chlorine, bromine, and iodine. R is preferably a halogen atom, more preferably chlorine, in view of anti-hepatitis B virus activity.

### Production Method in Which R is a Halogen Atom in Formula (1)

The method of producing a compound in which R is a halogen atom in formula (1) is not limited. For example, the method comprises step A of reacting a compound represented by the following formula (I): 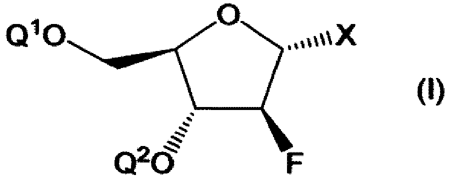
wherein Q¹ and Q² are the same or different, and each represents a protecting group of a hydroxyl group, and X represents bromine or iodine,
with 2-amino-6-haropurin, and step B of deprotecting the protecting group of the hydroxyl group of the product obtained by the reaction of step A.

### Step A

In formula (I), Q¹ and Q² are not limited as long as they are functional groups capable of protecting the hydroxyl group. Examples include ether-type protecting groups (e.g., t-butyl, benzyl, and trityl), acetal-type protecting groups (e.g., tetrahydropyranyl), acyl-type protecting groups (e.g., acetyl and benzoyl), and silyl ether-type protecting groups (e.g., t-butyldimethylsilyl).

The amount of the compound represented by formula (I) to be used and the amount of 2-amino-6-haropurin to be used are not limited as long as the reaction proceeds. The molar ratio of the compound represented by formula (I) and 2-amino-6-haropurin can be in the range of 1:1 to 1:5.

The reaction of step A is usually carried out in the presence of a base. The base is preferably a non-nucleophilic base, and examples include metal alkoxides (e.g., sodium t-butoxy and potassium t-butoxy).

The reaction of step A is usually performed in a solvent.

Examples of the solvent include halogen-based solvents (e.g., dichloromethane, chloroform, and dichloroethane), alcohol-based solvents (e.g., ethanol, propanol, butanol, and pentanol), nitrile-based solvents (e.g., acetonitrile), and mixtures of these solvents.

The reaction temperature of step A is not limited as long as the reaction proceeds. It is, for example, in the range of 15 to 80°C.

The reaction time of step A can be set such that the target product is sufficiently obtained, and step A can be continued until the reaction is completed.

### Step B

The method and conditions for deprotecting the protecting group of the hydroxyl group of the product obtained by the reaction of step A can be suitably selected according to the type of the protecting group. For example, a benzoyl group can be deprotected by a reaction with a metal alkoxide (such as sodium methoxide).

The reaction temperature of step B is not limited as long as the reaction proceeds. It is, for example, in the range of 15 to 80°C.

The reaction time of step B can be set such that the target product is sufficiently obtained, and step B can be continued until the reaction is completed.

The method of producing the compound represented by formula (I) is not limited. For example, the method comprises the step of reacting a compound represented by the following formula (II) : 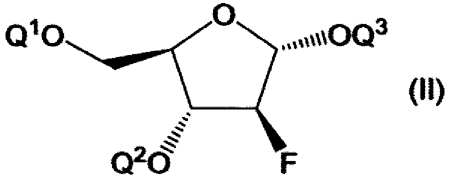
wherein Q¹ and Q² are each as defined above, and Q³ represents a protecting group of a hydroxyl group,
with hydrogen bromide or hydrogen iodide.

The amount of the compound represented by formula (II) to be used, and the amount of hydrogen bromide or hydrogen iodide to be used are not limited as long as the reaction proceeds. The molar ratio of the compound represented by formula (II) and hydrogen bromide or hydrogen iodide is, for example, in the range of 1:1 to 1:5.

The reaction of the compound represented by formula (II) and hydrogen bromide or hydrogen iodide is usually performed in a solvent.

Examples of the solvent include halogen-based solvents (e.g., dichloromethane, chloroform, and dichloroethane), carboxylic acid-based solvents (e.g., acetic acid), and mixtures of these solvents.

The reaction temperature is not limited as long as the reaction proceeds, and is, for example, in the range of 15 to 30°C.

2-Amino-6-haropurin may be a commercially available product or a synthetic product. The method for synthesizing 2-amino-6-fluoropurine is not limited; however, it may comprise step A1 of reacting 2-amino-6-chloropurine with trialkyl amine, and step A2 of reacting the product obtained by the reaction with a hydrogen fluoride salt.

### Step A1

Examples of trialkylamines include tri(C₁₋₄ alkyl)amines such as trimethylamine and triethylamine. The amount of 2-amino-6-chloropurine to be used, and the amount of trialkylamine to be used are not limited as long as the reaction proceeds. The molar ratio of 2-amino-6-chloropurine and trialkylamines is, for example 1:1 to 1:100, and more preferably 1: 2 to 1:50.

The reaction of step A1 is usually carried out in a solvent. Examples of the solvent include water, ketone solvents (e.g. acetone and methylethyl ketone), amide solvents (e.g., dimethylformamide and dimethylacetamide), and mixtures of these solvents.

The reaction temperature of step A1 is not limited as long as the reaction proceeds; however, it may be in the range of 15 to 80°C.

### Step A2

Examples of the hydrogen fluoride salt include alkali metal salts of hydrogen fluoride, such as hydrogen fluoride lithium, sodium hydrogen fluoride, and potassium hydrogen fluoride.

The amount of the product obtained by the reaction of step A1 to be used and the amount of hydrogen fluoride salt to be used are not particularly limited as long as the reaction proceeds. The molar ratio of the product obtained by the reaction of step A1 and hydrogen fluoride salt is, for example, 1:1 to 1:100, preferably 1:2 to 1:50, and more preferably 1:3 to 1:10.

The reaction of step A2 is usually carried out in a solvent. Examples of the solvent include water, ketone solvents (e.g. acetone and methylethyl ketone), amide solvents (e.g., dimethylformamide and dimethylacetamide), and mixtures of these solvents.

The reaction temperature of step A2 is not limited as long as the reaction proceeds. The reaction of step A2 can proceed by heating. The reaction temperature of step A2 is, for example, in the range of 15 to 150°C, and preferably 30 to 120°C.

### Production Method of Compound in Which R is an Amino Group in Formula (1)

The production method of a compound in which R is an amino group in formula (1) is not limited. For example, the method includes step C of reacting the compound in which R is a halogen atom in formula (1) with ammonia.

The amount of the compound in which R is a halogen atom in formula (1) to be used, and the amount of ammonia to be used are not limited as long as the reaction proceeds. The molar ratio of the compound in which R is a halogen atom in formula (1) and ammonia is, for example, in the range of 1:3 to 1:200, and preferably 1:5 to 1:100.

The reaction of step C is usually carried out in a solvent. Examples of such solvents include hydrocarbon solvents (e.g. hexane, toluene, and xylene), alcohol-based solvents (e.g., methanol and ethanol), and mixtures of these solvents.

The reaction temperature of step C is not limited as long as the reaction proceeds. It is, for example, in the range of 15 to 80°C.

The reaction time of step C can be set such that the target product is sufficiently obtained, and step C can be continued until the reaction is completed.

### Production Method of Compound in Which R is a Methoxy Group in Formula (1)

The production method of a compound in which R is a methoxy group in formula (1) is not limited. For example, the method includes step D of reacting the compound in which R is a halogen atom in formula (1) with methanol.

The amount of the compound in which R is a halogen atom in formula (1) to be used and the amount of methanol to be used are not limited as long as the reaction proceeds. The molar ratio of the compound in which R is a halogen atom in formula (1) and methanol is, for example, in the range of 1:1 to 1:100. Methanol can be used as a solvent.

The reaction temperature of step D is not limited as long as the reaction proceeds. It is, for example, in the range of 15 to 80°C.

The reaction time of step D can be set such that the target product is sufficiently obtained, and step C can be continued until the reaction is completed.

### Production Method of Compound in Which R is a Cyano Group in Formula (1)

The production method of a compound in which R is a cyano group in formula (1) is not limited. For example, the method includes step E of reacting the compound in which R is a halogen atom in formula (1) with a metal cyanide.

In step E, examples of the metal cyanide include copper cyanide, potassium cyanide, sodium cyanide, zinc cyanide, and the like.

The amount of the compound in which R is a halogen atom in formula (1) to be used, and the amount of the metal cyanide to be used are not limited as long as the reaction proceeds. The molar ratio of the compound in which R is a halogen atom in formula (1) and the metal cyanide is, for example, in the range of 1:1 to 1:100, and preferably 1:5 to 1:50.

The reaction of step E is usually carried out in a solvent. Examples of such solvents include alcohol-based solvents (e.g., methanol and ethanol), amine-based solvents (e.g., pyridine), and mixtures of these solvents.

The reaction temperature of step E is not limited as long as the reaction proceeds. It is, for example, in the range of 15 to 80°C.

The reaction time of step E can be set such that the target product is sufficiently obtained, and step E can be continued until the reaction is completed.

The product obtained by the reaction in each step can be purified by a technique such as filtration and column chromatography, as desired.

### Prodrug

The prodrug of the compound represented by formula (1) is not limited as long as it can be converted to its active metabolite or the compound represented by formula (1) *in vivo.* Any prodrug that is used as a prodrug of a nucleic acid analogue can be used.

Typical examples of the prodrug include esters and ester amides.

Examples of the esters include phosphoric acid esters. Preferable example include a phosphoric acid monoester represented by the following formula: 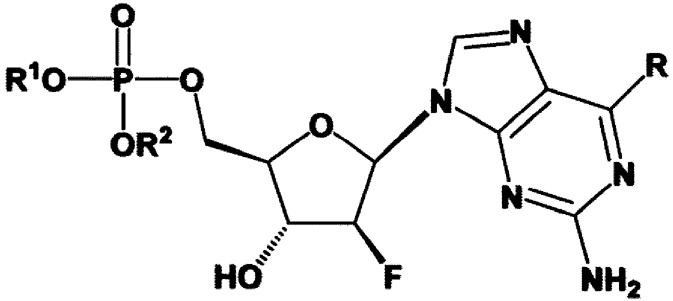 wherein R is as defined above, R¹ and R² are the same or different, and each represents a hydrogen atom, an alkyl group optionally having one or more substituents, a cycloalkyl group optionally having one or more substituents, an aryl group optionally having one or more substituents, or an aralkyl group optionally having one or more substituents; or R¹ and R² are bonded each other to form a ring together with phosphorus and oxygen atoms constituting a phosphoric acid ester moiety in the structural formula; and a phosphoric acid di- or tri-ester represented by the following formula: 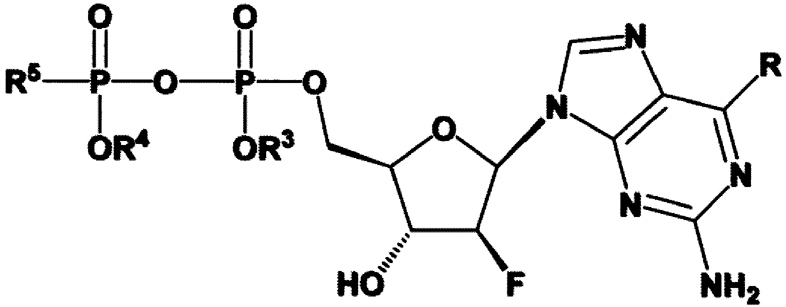
wherein R is as defined above, R³ and R⁴ in each occurrence are the same or different, and each represents a hydrogen atom, an alkyl group optionally having one or more substituents, a cycloalkyl group optionally having one or more substituents, an aryl group optionally having one or more substituents, or an aralkyl group optionally having one or more substituents;
R⁵ represents an alkyl group optionally having one or more substituents, an alkoxy group optionally having one or more substituents, an alkoxyalkoxy group optionally having one or more substituents, an acyloxy group optionally having one or more substituents, or a steroid group optionally having one or more substituents (a group containing cyclopentaphenanthrene or hydrogenated cyclopentaphenanthrene); and
n represents 1 or 2.

Examples of the alkyl group in the present specification include linear or branched C₁₋₂₀ alkyl (e.g., methyl, ethyl, propyl (n-propyl and i-propyl), butyl (n-butyl, s-butyl, i-butyl, and t-butyl), pentyl, hexyl, heptyl, octyl, 2-ethylhexyl, nonyl, decyl, undecyl, dodecyl, tridecyl, tetradecyl, pentadecyl, hexadecyl, heptadecyl, and octadecyl).

Examples of the cycloalkyl group in the present specification include C₃₋₁₀ cycloalkyl (e.g., cyclopentyl and cyclohexyl).

Examples of the aryl group in the present specification include C₆₋₁₀ aryl (e.g., phenyl and naphthyl) .

Examples of the aralkyl group in the present specification include C₆₋₁₀ aryl-linear or branched C₁₋₁₀ alkyl (e.g., benzyl and phenethyl).

Examples of the alkoxy group in the present specification include linear or branched C₁₋₁₀ alkyloxy (e.g., methoxy, ethoxy, and propoxy).

Examples of the alkoxyalkoxy group in the present specification include linear or branched C₁₋₂₀ alkyloxy, linear or branched C₁₋₄ alkyloxy (e.g., methoxymethoxy, methoxyethoxy, and ethoxyethoxy, hexadecyloxypropoxy, and octadecyloxyethoxy).

Examples of the acyloxy group in the present specification include linear or branched C₁₋₁₀ alkylcarbonyloxy (e.g., methylcarbonyloxy, ethylcarbonyloxy, and propylcarbonyloxy) .

Examples of substituents that each of the alkyl, cycloalkyl, aryl, aralkyl, alkoxy, alkoxyalkoxy, acyloxy, and steroid groups may have include halogens and organic groups. Preferable examples include fluorine, chlorine, bromine, alkoxy, alkylcarbonyloxy, alkylcarbonylthio, alkyloxycarbonyl, and alkyldithio. Most preferable examples include halogens, linear or branched C₁₋₂₀ alkyloxy, linear or branched C₁₋₁₀ alkylcarbonyloxy, linear or branched C₁₋₁₀ alkylcarbonylthio, linear or branched C₁₋₁₀ alkyloxycarbonyl, and linear or branched C₁₋₁₀ alkyldithio.

R¹ preferably represents an alkyl group optionally having one or more substituents, and more preferably an alkyl group optionally having one or more substituents selected from the group consisting of alkoxy, alkylcarbonyloxy, alkylcarbonylthio, and alkyldithio.

R² preferably represents a hydrogen atom, an alkyl group optionally having one or more substituents, an aryl group optionally having one or more substituents, or an aralkyl group optionally having one or more substituents; and more preferably a hydrogen atom, an alkyl group, an aryl group, or an aralkyl group.

The ring that is formed by binding R¹ and R² each other, together with a phosphorus atom and an oxygen atom constituting a phosphoric acid ester moiety, can be a monocyclic ring or a fused ring.

The number of constituent atoms of the ring is, for example, an integer in the range of 6 to 10.

Examples of the ring include rings represented by the following formulae:

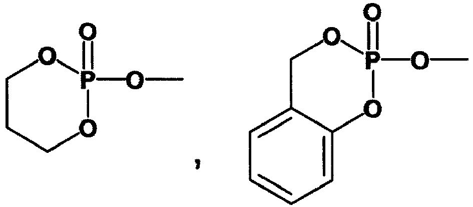

The ring optionally has one or more substituents. Examples of the substituent include halogen, alkyl, cycloalkyl, aryl, and aralkyl.

R³ and R⁴ preferably represent a hydrogen atom, an alkyl group optionally having one or more substituents, an aryl group optionally having one or more substituents, or an aralkyl group optionally having one or more substituents; and even more preferably a hydrogen atom, an alkyl group, an aryl group, or an aralkyl group.

R⁵ preferably represents an alkyl group, an alkoxy group, an alkoxyalkoxy group, an acyloxy group, or a steroid group.

Specific examples of the ester amide include phosphoric acid ester amides. Preferable examples include a compound represented by the following formula: 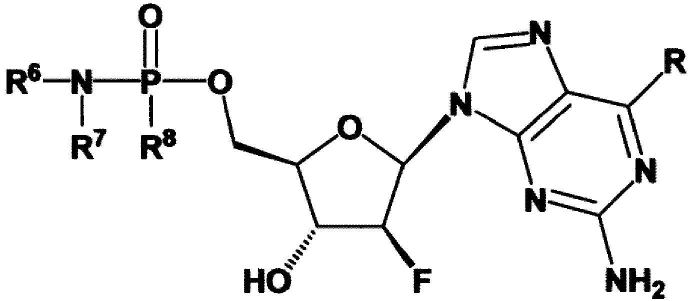 wherein
R is as defined above,
R⁸ represents -NR^{8a}R^{8b} or -OR^{8c}; and
R⁶, R⁷, R^{8a}, R^{8b}, and R^{8c} are the same or different, and each represents a hydrogen atom, an alkyl group optionally having one or more substituents, a cycloalkyl group optionally having one or more substituents, an aryl group optionally having one or more substituents, or an aralkyl group optionally having one or more substituents.

R⁶ and R^{8a} preferably represent an alkyl group optionally having one or more substituents, and more preferably an alkyl group optionally having a substituent selected from the group consisting of halogen and alkyloxycarbonyl.

R⁷ and R^{8b} preferably represent a hydrogen atom or an alkyl group optionally having one or more substituents, and even more preferably a hydrogen atom or an alkyl group.

R^{8c} preferably represents a hydrogen atom, an alkyl group optionally having one or more substituents, an aryl group optionally having one or more substituents, an aryl group optionally having one or more substituents, an aralkyl group optionally having one or more substituents; and more preferably a hydrogen atom, an alkyl group, an aryl group, or an aralkyl group.

More preferable examples of the prodrug include compounds represented by the following formulae:

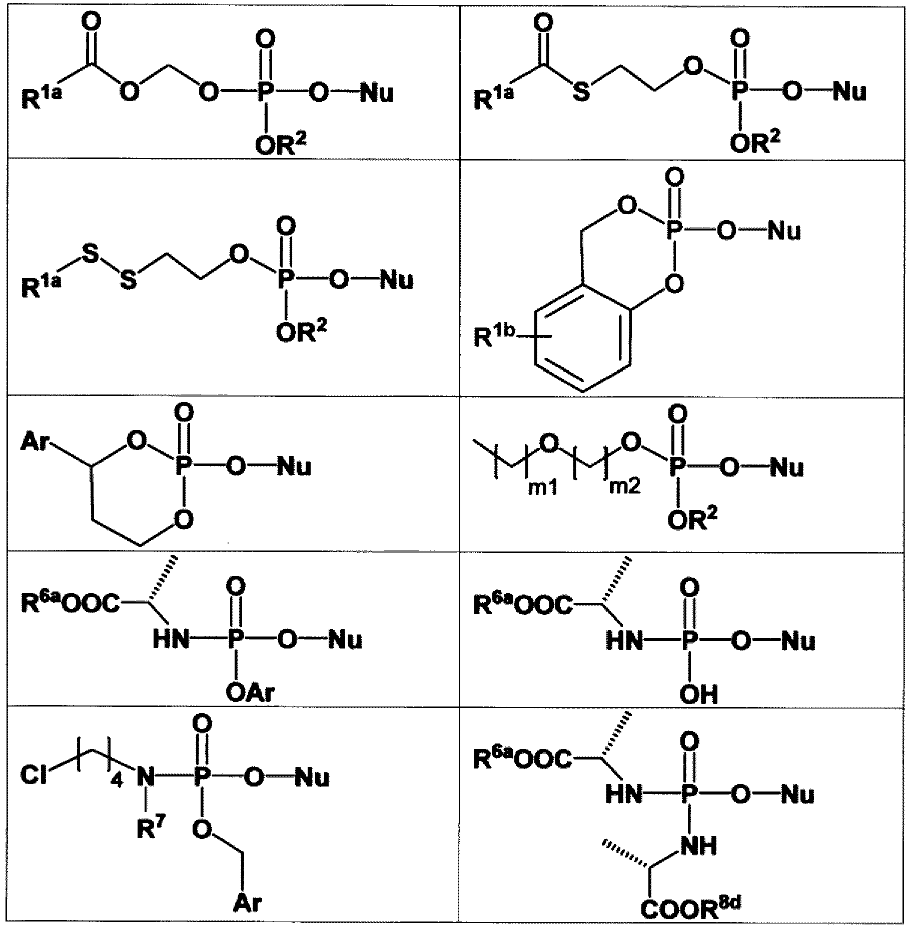

wherein
R^{1a}, R^{6a}, and R^{8d} each represent an alkyl group;
R^{1b} represents halogen or an alkyl group;
R² and R⁷ are as defined above;
Ar represents an aryl group; and
Nu represents a group represented by the following formula: 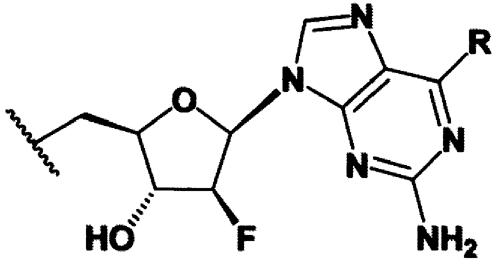
   wherein R is as defined above,
   m1 represents an integer in the range of 1 to 18, and
   m2 represents an integer in the range of 1 to 10.

The prodrug can be produced according to its chemical structure based on technical knowledge with reference to a known method (e.g., a method described in Chemical Reviews 2014, vol. 114, pp. 9154-9218).

### Salt

Examples of the pharmaceutically acceptable salt of the compound represented by formula (1) or its prodrug include (1) salts with an inorganic acid (e.g., hydrochloric acid, sulfuric acid, phosphoric acid, hydrobromic acid, hydriodic acid, nitric acid, pyrosulfuric acid, and metaphosphoric acid), (2) salts with an organic acid (e.g., citric acid, benzoic acid, acetic acid, propionic acid, fumaric acid, maleic acid, and sulfonic acid (e.g., methanesulfonic acid, p-toluenesulfonic acid, and naphthalenesulfonic acid)), and (3) alkali metal salts (e.g., sodium salts and potassium salts).

### Solvate

Examples of the solvates of the compound represented by formula (1) or its prodrug, or a pharmaceutically acceptable salt thereof include hydrates and organic solvates (e.g., methanol solvates, ethanol solvates, and dimethyl sulfoxide solvates).

### Other Active Ingredients

The anti-hepatitis B virus agent may further contain other active ingredients.

Examples of "other active ingredients" include other nucleic acid analogues (such as 2'-deoxy-2'-fluoro-nucleoside), and other anti-hepatitis B virus agents.

Two or more anti-hepatitis B virus agents may be used.

The compound represented by formula (1) or its prodrug, or a pharmaceutically acceptable salt thereof, or a solvate thereof may be each formulated into a formulation separated from other active ingredients.

The compound represented by formula (1) or its prodrug, or a pharmaceutically acceptable salt thereof, or a solvate thereof may be administered simultaneously, sequentially, or alternately with other active ingredients.

### Amount of Active Ingredient

The lower limit of the content of the active ingredient can be set to, for example, 0.001 mass%, preferably 0.01 mass%, and more preferably 0.05 mass%, relative to the total mass of the anti-hepatitis B virus agent, in terms of activity. The upper limit of the content of the active ingredient is not limited, and can be set to, for example, 99.99 mass%, preferably 90 mass%, and more preferably 80 mass%, relative to the total mass of the anti-hepatitis B virus agent. The amount of the active ingredient is in the range in which the lower limit and the upper limit are arbitrary selected. For example, it is in the range of 0.001 to 99.99 mass%, preferably 0.01 to 90 mass%, and more preferably 0.05 to 80 mass%.

### Additive

The anti-hepatitis B virus agent may include a pharmaceutically acceptable additive.

Examples of the form of the anti-hepatitis B virus agent include solid formulations (e.g., granules, sprays, tablets, capsules, and dry syrups), semi-solid formulations (e.g., creams, ointments, and gels), and liquid formulations (e.g., solutions and suspensions).

The solid formulation can be produced, for example, by mixing an active ingredient and an additive (e.g., an excipient, binder, disintegrant, lubricant, and colorant), and if necessary, by granulation, particle size regulation, compression, and/or coating.

Examples of the excipient include lactose, lactose hydrate, sucrose, mannitol, sorbitol, crystalline cellulose, starch (e.g., cornstarch), hydrous silicon dioxide, and combinations thereof.

Examples of the binder include agar, gum arabic, hyaluronic acid, hydroxyethyl cellulose, hydroxypropyl cellulose, hydroxypropyl methyl cellulose, and combinations thereof.

Examples of the disintegrant include alginic acid, carboxymethyl cellulose (carmellose), croscarmellose sodium, low substituted hydroxypropyl cellulose, polyvinylpyrrolidone (povidone), crospovidone, and combinations thereof.

Examples of the lubricant include stearic acid, magnesium stearate, calcium stearate, talc, and combinations thereof.

Examples of the colorant include iron trioxide, titanium oxide, and combinations thereof.

The semi-solid formulation can be produced, for example, by mixing an active ingredient, a semi-solid carrier, and optionally other additives.

The liquid formulation can be produced, for example, by mixing an active ingredient, a liquid carrier (e.g., aqueous carrier (e.g., purified water), and an oily carrier), and optionally other additives (e.g., an emulsifier, dispersant, suspending agent, buffer, antioxidant, surfactant, osmotic pressure regulator, chelating agent, and antimicrobial agent), and by sterilizing, as necessary.

### Form of Administration

The method of administering the anti-hepatitis B virus agent includes oral or non-oral administration (e.g., intravenous, intramuscular, or subcutaneous administration).

The anti-hepatitis B virus agent may be topically administered.

The anti-hepatitis B virus agent may be administered to humans, non-human mammals (e.g., monkeys, sheep, dogs, mice, and rats), and non-mammals.

The number of administrations of the anti-hepatitis B virus agent can be selected according to the age, weight, medical condition, etc. of the subject. The anti-hepatitis B virus agent can be administered, for example, once, twice, or three times a day; once every two days; once every three days; or once a week.

The single dose of the anti-hepatitis B virus agent may range from 0.1 mg to 1000 mg, depending on the target of administration and the frequency of administration.

Preferable examples of the anti-hepatitis B virus agent include orally administered formulations. Examples include a tablet containing an active ingredient, crystalline cellulose, hydroxypropyl methylcellulose, povidone, magnesium stearate, and titanium oxide; and a hard gelatin capsule containing an active ingredient, povidone, and magnesium stearate.

### Prophylactic or Therapeutic Agent for Hepatitis B Virus-related Diseases

The term "hepatitis B virus-related disease" means a disease that occurs as a result of infection with hepatitis B virus. The hepatitis B virus-related disease can be at least one member selected from the group consisting of hepatitis B (acute hepatitis B and chronic hepatitis B), type-B liver cirrhosis, and type-B liver cancer.

The prophylactic or therapeutic agent for a hepatitis B virus-related disease according to one embodiment of the present disclosure comprises, as an active ingredient, the compound represented by formula (1) or its prodrug, or a pharmaceutically acceptable salt thereof, or a solvate thereof.

The dosage form and the administration form (e.g., administration route, administration target, administration frequency, and dosage) of other active ingredients and additives that can be contained in the prophylactic or therapeutic agent, and the prophylactic or therapeutic agent can be selected from those mentioned for the anti-hepatitis B virus agent described above.

### Method of Delaying or Inhibiting Growth of Hepatitis B Virus, or Method of Preventing or Treating Hepatitis B Virus-related Disease

The method of delaying or inhibiting the growth of hepatitis B virus, or the method of preventing or treating a hepatitis B virus-related disease according to one embodiment of the present disclosure includes the step of administering, to a subject, the compound represented by formula (1) or its prodrug, or a pharmaceutically acceptable salt thereof, or a solvate thereof, as necessary.

The administration form (e.g., administration route, administration target, administration frequency, and dosage) can be selected from those described for the anti-hepatitis B virus agent described above.

### Examples

One embodiment of the present disclosure is described in more detail by means of examples; however, the present disclosure is not limited to these.

### Example 1: Synthetic Example

(1) 2 g of 2-deoxy-2-fluoro-1,3,5-tri-O-benzoyl-α-D-arabinofuranose was dissolved in 20 mL of dichloromethane, and 4 mL of a hydrogen bromide acetic acid solution (5.1 mol/L) was added thereto, followed by stirring. After completion of the reaction, a saturated sodium bicarbonate aqueous solution was added to neutralize the unreacted HBr.

Thereafter, separation was conducted, and the solution extracted with dichloromethane was concentrated, thereby obtaining 1.7 g of a crude of 1-bromo-2-deoxy-2-fluoro-3,5-di-O-benzoyl-α-D-arabinofuranose, which was used in the next step.

### (2) Reaction with 2-amino-6-chloropurine

1 g of 2-amino-6-chloropurine was dispersed in a cosolvent of t-amyl alcohol:acetonitrile.

Potassium t-butoxy was added thereto, and the mixture was heated to 50°C, stirred for a while, and dissolved.

1.7 g of a crude of 1-bromo-2-deoxy-2-fluoro-3,5-di-O-benzoyl-α-D-arabinofuranose that had been dissolved in acetonitrile was added thereto dropwise and subjected to a reaction.

After completion of the reaction, the solid component was diluted with dichloroethane and subjected to filtration. Thereafter, the filtrate was concentrated and purified by column chromatography.

210 mg of a reaction product (9-(2'-deoxy-2'-fluoro-3',5'-di-O-benzoyl-β-D-arabinofranos-1'-yl)-2-amino-6-chloropurine) was obtained.

### (3) Deprotection

100 mg of 9-(2'-deoxy-2'-fluoro-3',5'-di-O-benzoyl-β-D-arabinofranos-1'-yl)-2-amino-6-chloropurine was dissolved in methanol, and 10 mg of sodium methoxide was added thereto, followed by stirring.

After completion of the reaction, the reaction product was purified by preparative TLC, thereby obtaining 60 mg of 9-(2'-deoxy-2'-fluoro-β-D-arabinofuranos-1'-yl)-2-amino-6-chloropurine (the following formula). 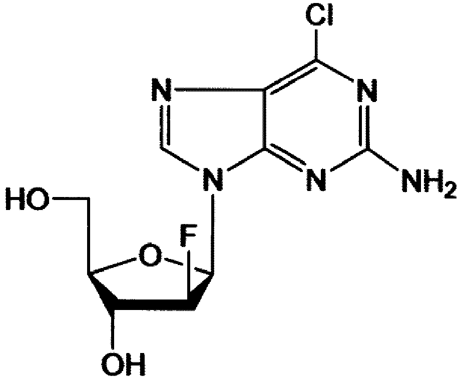
¹H NMR (methanol-d₄): 8.21 (d, 1H), 6.50 and 6.31 (dd, 1H), 5.14 (t, 0.5H), 5.01 (t, 0.5H), 4.50 (t, 0.5H), 4.45 (t, 0.5H), 4.00 to 3.90 (m, 1H), 3.85 to 3.75 (m, 2H);
¹⁹F NMR (methanol-d₄): -199.15 to -199.41 (m)

### Example 2: Synthetic Example

### (1) Synthesis of 2-amino-6-fluoropurine

1.5 g of 2-amino-6-chloropurine was added to 50 mL of dimethylformamide (DMF), and the mixture was stirred at 70°C for 1 hour and cooled to room temperature.

4.0 mL of a trimethylamine aqueous solution was added thereto, followed by stirring for the reaction.

After completion of the reaction, filtration was conducted, and the precipitate was washed with DMF and acetone, and then dried, thus obtaining 1.5 g of 2-amino-N, N, N-trimethyl-9H-purin-6-aminium chloride.

0.5 g of 2-amino-N, N, N-trimethyl-9H-purin-6-aminium chloride and 0.5 g of potassium hydrogen fluoride were placed in 50 mL of DMF, followed by stirring at 90°C.

After concentration, 0.12 g of 2-amino-6-fluoropurine was obtained by subjecting the residue to flash chromatography.

(2) The same operation as in step (2) of Example 1 was performed except that 2-amino-6-fluoropurine was used in place of 2-amino-6-chloropurine, thus obtaining 0.13 g of 9-(2'-deoxy-2'-fluoro-3',5'-di-O-benzoyl-β-D-arabinofranos-1'-yl)-2-amino-6-fluoropurine from 0.32 g of a crude of 1-bromo-2-deoxy-2-fluoro-3,5-di-O-benzoyl-α-D-arabinofuranose.

### (3) Deprotection

The same operation as in step (3) of Example 1 was performed except that 100 mg of 9-(2'-deoxy-2'-fluoro-3',5'-di-O-benzoyl-β-D-arabinofranos-1'-yl)-2-amino-6-fluoropurine was used in place of 9-(2'-deoxy-2'-fluoro-3',5'-di-O-benzoyl-β-D-arabinofranos-1'-yl)-2-amino-6-chloropurine, thus obtaining 65 mg of 9-(2'-deoxy-2'-fluoro-β-D-arabinofuranos-1'-yl)-2-amino-6-fluoropurine (following formula). 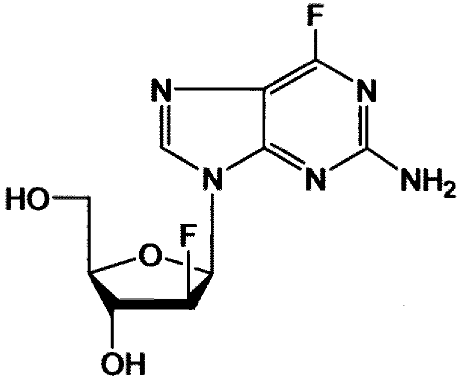
¹H NMR (acetone-d₆): 7.74 (d, 1H), 6.34 and 6.30 (dd, 1H), 5.33 (br s, 2H), 5.18 (t, 0.5H), 5.06 (t, 0.5H), 4.67 (t, 0.5H), 4.62 (t, 0.5H), 3.95 to 3.90 (m, 1H), 3.85 to 3.70 (m, 2H);
¹⁹F NMR (acetone-d₆): -72.98 (s), -198.68 to -199.05 (m)

### Example 3: Synthetic Example

The same operation as in step (2) of Example 1 was performed except that 2-amino-6-iodopurine was used in place of 2-amino-6-chloropurine, thus obtaining 0.11 g of 9-(2'-deoxy-2'-fluoro-3',5'-di-O-benzoyl-β-D-arabinofranos-1'-yl)-2-amino-6-iodopurine from 0.35 g of a crude of 1-bromo-2-deoxy-2-fluoro-3,5-di-O-benzoyl-α-D-arabinofuranose.

The same operation as in step (3) of Example 1 was performed except that 110 mg of 9-(2'-deoxy-2'-fluoro-3',5'-di-O-benzoyl-β-D-arabinofranos-1'-yl)-2-amino-6-iodopurine was used in place of 9-(2'-deoxy-2'-fluoro-3',5'-di-O-benzoyl-β-D-arabinofranos-1'-yl)-2-amino-6-chloropurine, thus obtaining 59 mg of 9-(2'-deoxy-2'-fluoro-β-D-arabinofuranos-1'-yl)-2-amino-6-iodopurine (following formula). 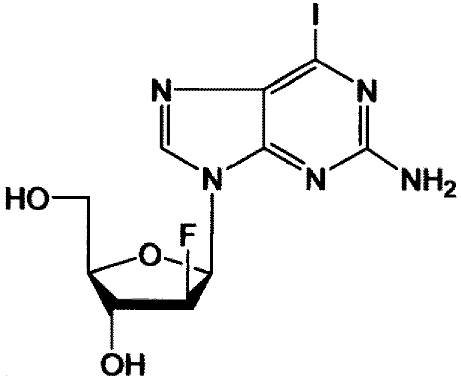
¹H NMR (methanol-d₄): 8.21 (d, 1H), 6.35 and 6.31 (dd, 1H), 5.14 (t, 0.5H), 5.01 (t, 0.5H), 4.50 (t, 0.5H), 4.45 (t, 0.5H), 3.90 to 3.94 (m, 1H), 4.00 to 3.90 (m, 1H), 3.85 to 3.70 (m, 2H);
¹⁹F NMR (methanol-d₄): α: -190.14 to -190.37 (m), -199.07 to -199.40 (m)

### Example 4: Synthetic Example

Ammonia (methanol solution) was added to 9.2 mg of 9-(2'-deoxy-2'-fluoro-3',5'-di-O-benzoyl-β-D-arabinofranos-1'-yl)-2-amino-6-fluoropurine obtained in Example 2, followed by stirring. The concentrated residue was purified by column chromatography to obtain 1.1 mg of 9-(2'-deoxy-2'-fluoro-β-D-arabinofuranos-1'-yl)-2-amino-6-aminopurine (following formula). 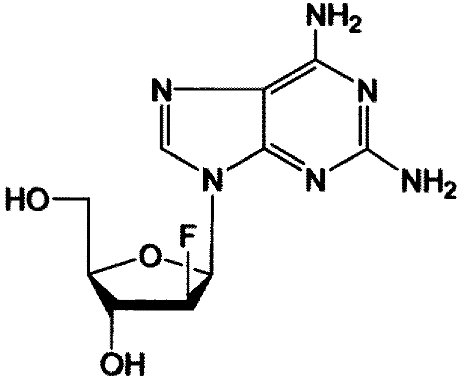
¹H NMR (acetone-d₆): 7.75 (d, 1H), 6.31 to 6.26 (m, 3H), 5.35 (br s, 2H), 5.19 (t, 0.5H), 5.19 (br s, 0.8 H), 5.06 (t, 0.5H), 4.69 (m, 1H), 4.67 (br s, 1H), 3.95 to 3.94 (m, 1H), 3.90 to 3.70 (m, 2H) ;
¹⁹F NMR (acetone-d₆): α: -183.01 to -183.26 (m), β:-198.73 to - 198.96 (m)

### Example 5: Synthetic Example

The same operation as in Example 4 was performed, thus obtaining 2.5 mg of 9-(2'-deoxy-2'-fluoro-β-D-arabinofuranos-1'-yl)-2-amino-6-methoxypurine (following formula). 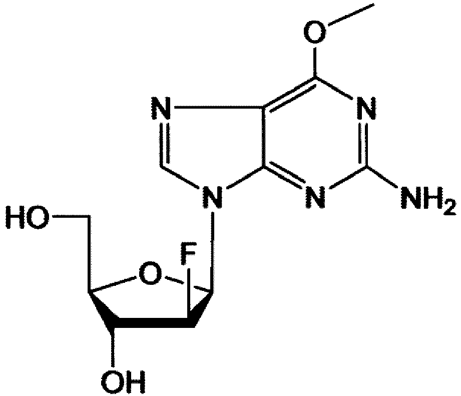
¹H NMR (acetone-d₆): 7.92 (d, 1H), 6.37 and 6.30 (dd, 1H), 5.88 (br s, 2H), 5.23 (t, 0.5H), 5.10 (t, 0.5H), 4.66 (t, 0.5H), 4.63 (t, 0.5H), 4.00 to 3.95 (m, 1H), 3.90 to 3.70 (m, 2H), 3.29 (s, 3H);
¹⁹F NMR (acetone-d₆): -198.34 to -199.01 (m)

### Example 6: Synthetic Example

0.06 g of 9-(2'-deoxy-2'-fluoro-β-D-arabinofuranos-1'-yl)-2-amino-6-chloropurine obtained in Example 1 was dissolved in 10 mL of pyridine, and then 0.068 g of copper cyanide was placed, and the mixture was heated with stirring.

After completion of the reaction, the mixture was concentrated and dissolved by adding 10 mL of methanol.

After the methanol solution was subjected to filtration, the resultant was concentrated to dryness, and the residue was purified by column chromatography, thus obtaining 0.0193 g of 9-(2'-deoxy-2'-fluoro-β-D-arabinofuranos-1'-yl)-2-amino-6-cyanopurine (following formula). 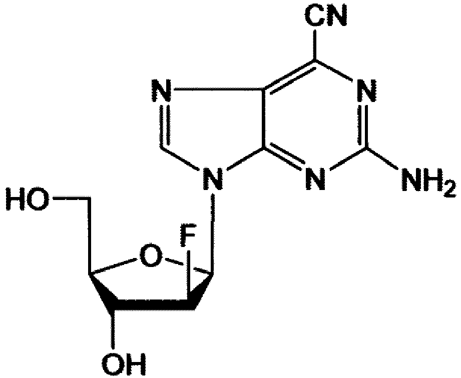 ¹H NMR (acetone-d₆): 8.36 (d, 1H), 6.46 (br s, 2H), 6.42 and 6.38 (dd, 1H), 5.30 and 5.17 (dd, 1H), 4.68 and 4.63 (dd, 1H), 4.01 (m, 1H), 3.83 (m, 2H); ¹⁹F NMR (acetone-d₆): -198.90 to -199.20 (m)

### Comparative Example 1: Synthetic Example

In step (2) of Example 1, the same operation was repeated as in Example 1, except that 2,6-dichloropurine was used in place of 2-amino-6-chloropurine to obtain 2,6-dichloro-9-((2R,3S,4R,5R)-3-fluoro-4-hydroxy-5-(hydroxymethyl) tetrahydrofuran-2-yl)-9H-purine (the following formula). 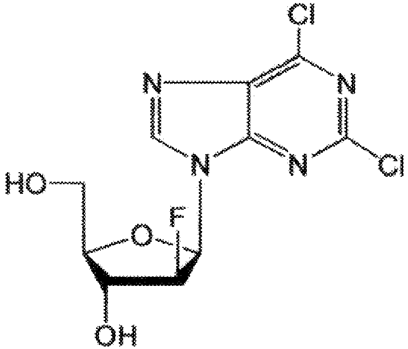 1H NMR (400 MHz, DMSO-D6) δ 8.53 (s, 1H), 6.41 (dd, J = 12.3, 4.6 Hz, 1H), 5.97 (s, 1H), 5.25 (dt, J = 52.8, 4.6 Hz, 1H), 5.09 (t, J = 5.5 Hz, 1H), 4.38-4.43 (m, 1H), 3.81-3.91 (m, 1H), 3.58-3.68 (m, 2H)

### Test Example 1: Anti-HBV Activity

Anti-HBV activity was evaluated by quantitative PCR for determining the amount of HBV DNA in cells on the seventh day after the addition of the agent, using the HepG2.2.15 cell line of HBV-producing cells obtained by introducing a gene having a length twice that of the HBV genome into the human liver cancer cell line HepG2. For PCR of HBV DNA, a forward primer (HBV-S190F; 5'-GCT CGT GTT ACA GGC GGG-3': SEQ ID NO: 1) and a reverse primer (HBV-S703R; 5'-GAA CCA CTG AAC AAA TGG CAC TAG TA-3': SEQ ID NO: 2) were used. PCR was performed by carrying out a reaction at 95°C for 10 sec, 62°C for 10 sec, and 72°C for 30 sec for 35 cycles.

Specifically, HepG2.2.15 cells were seeded at a concentration of 1 × 10⁵ cells/well. 24 hours later, the compounds of Example 1 and Comparative Example 1 were diluted to have a concentration of 0, 0.49, 0.97, 1.95, 3.90, 7.81, 15.6, 31.3, 62.5, 125, 250, 500, and 1000 nM, and added to the cells. After the cells were cultured for 7 days, each cytoplasmic compartment was collected, and DNA was purified by phenol-chloroform extraction. 20 ng of the purified DNA was used to measure the amount of intracellular HBV DNA by quantitative PCR.

### Test Example 2: Cytotoxicity Test

HepG2 NTCP-myc cells were seeded at a concentration of 2 × 10⁴ cells/well. 24 hours later, the compound of Example 1 was diluted to have a concentration of 0, 0.49, 0.97, 1.95, 3.90, 7.81, 15.6, 31.3, 62.5, 125, 250, 500, and 1000 nM, and added to the cells. The HepG2 NTCP-myc cells were cultured for 7 days. After the culture, 10 µl of Premix WST-1 Cell Proliferation Assay System (TaKaRa) was added, and the cells were cultured at 37°C for 2 hours. Thereafter, absorbance was determined at 450 nm using a microplate reader.

### Results

### 1. Anti-HBV Activity

The EC₅₀ (50% effective concentration) was calculated from a graph showing the relationship between the concentration of the compound and anti-HBV activity. The EC₅₀ of each compound in the Examples and Comparative Examples is shown in Table 1.

**Table 1**

| | EC₅₀ |
|---|---|
| Example 1 | 11.8 nM |
| Example 2 | 0.88 µM |
| Example 3 | 0.99 µM |
| Example 4 | 2.02 µM |
| Example 5 | 1.63 µM |
| Example 6 | 0.93 µM |
| Comparative Example 1 | >10 µM |

The results of Table 1 indicate that the compounds of Examples 1 to 6 have high anti-HBV activity, as compared to the compound of Comparative Example 1.

### 2. Cytotoxicity

The CC₅₀ (50% cytotoxic concentration) was calculated from a graph showing the relationship between the concentration of the compound and cytotoxicity. The CC₅₀ of each compound in the Examples relative to HepG2 NTCP-myc cells is shown in Table 2.

**Table 2**

| | CC₅₀ |
|---|---|
| Example 1 | >500 µM |
| Example 2 | 337 µM |
| Example 3 | >500 µM |
| Example 4 | >500 µM |
| Example 5 | >500 µM |
| Example 6 | >500 µM |

The results of Table 2 indicate that the compounds of Examples 1 to 6 have low cytotoxicity.

### Sequence Listing

P20-050WO_PCT_anti-hepatitis B virus agent _20200312_113540_26.txt

## Claims

1. An anti-hepatitis B virus agent comprising, as an active ingredient, a compound represented by the following formula (1): 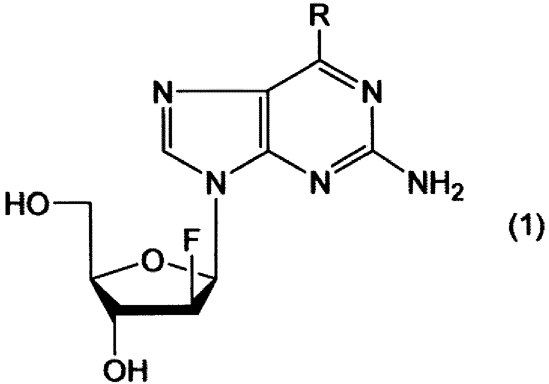
wherein R is a halogen atom, an amino group, a methoxy group, or a cyano group,
or its prodrug, or a pharmaceutically acceptable salt thereof, or a solvate thereof.

2. A prophylactic or therapeutic agent for a hepatitis B virus-related disease, comprising, as an active ingredient, a compound represented by the following formula (1): 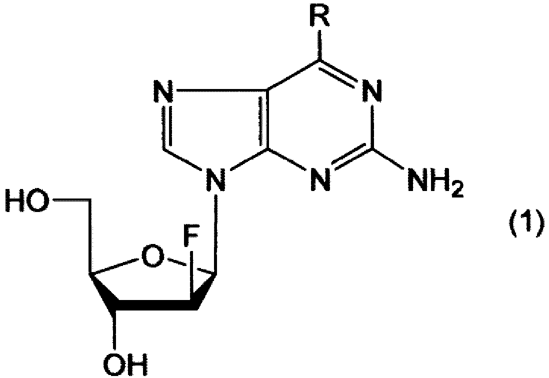
wherein R is a halogen atom, an amino group, a methoxy group, or a cyano group,
or its prodrug, or a pharmaceutically acceptable salt thereof, or a solvate thereof.

3. The prophylactic or therapeutic agent according to claim 2, wherein the hepatitis B virus-related disease is one or more diseases selected from the group consisting of hepatitis B, type-B liver cirrhosis, and type-B liver cancer.

4. A compound represented by the following formula (2) or (3) : 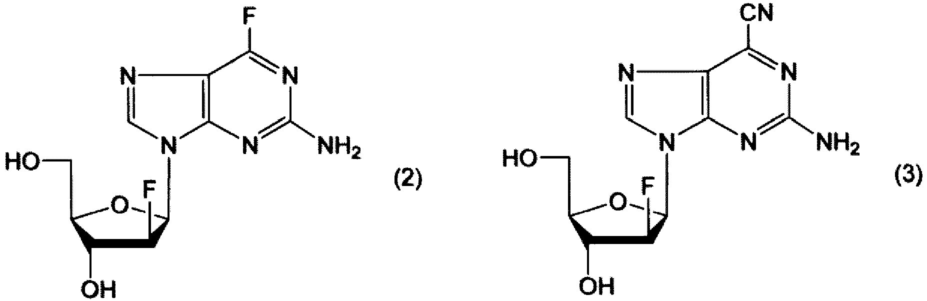 or its prodrug, or a pharmaceutically acceptable salt thereof, or a solvate thereof.

5. A method for producing a compound represented by the following formula (2): 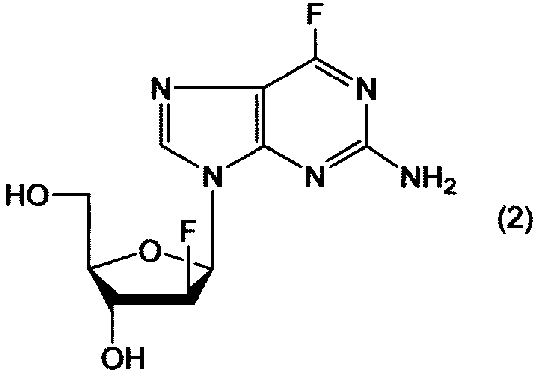 or its prodrug, or a pharmaceutically acceptable salt thereof, or a solvate thereof,
the method comprising
step A1 of reacting 2-amino-6-chloropurine with trialkylamine,
step A2 of reacting the product obtained by the reaction of step A1 with a hydrogen fluoride salt,
step A of reacting the product obtained by the reaction of step A2 with a compound represented by the following formula (I): 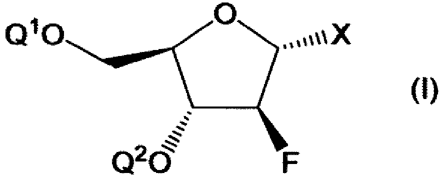
wherein Q¹ and Q² are the same or different, and each represents a protecting group of a hydroxyl group, and X is bromine or iodine, and
step B of deprotecting the protecting group of the hydroxyl group of the product obtained by the reaction of step A.

6. A method for producing a compound represented by the following formula (3): 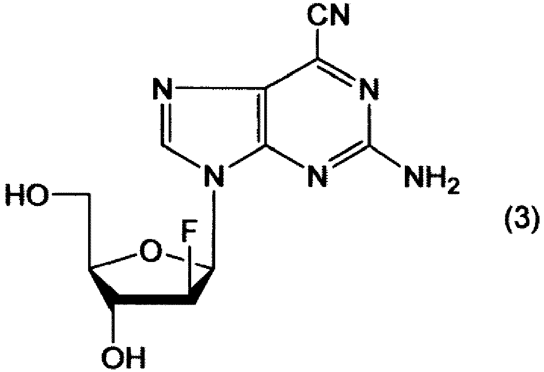
or its prodrug, or a pharmaceutically acceptable salt thereof, or a solvate thereof,
the method comprising reacting a compound represented by the following formula (4): 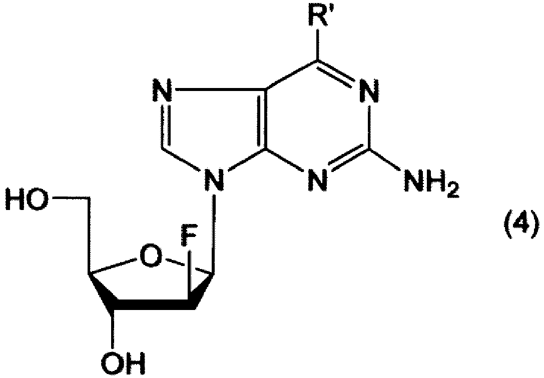
wherein R' is a halogen atom, with a metal cyanide.


SEQUENCE LISTING

<110>  DAIKIN INDUSTRIES, LTD.
       KAGOSHIMA UNIVERSITY

<120>  Anti-Hepatitis B Virus Agent

<130>  P20-050WO

<150>  JP 2019-046954
<151>  2019-03-14

<160>  2

<170>  PatentIn version 3.5

<210>  1
<211>  18
<212>  DNA
<213>  Artificial

<220>
<223>  primer

<400>  1
gctcgtgtta caggcggg                                                     18


<210>  2
<211>  26
<212>  DNA
<213>  Artificial

<220>
<223>  primer

<400>  2
gaaccactga acaaatggca ctagta                                            26